# EUROPEAN PATENT SPECIFICATION

(11) **EP 4 573 516 B1**
(45) Date of publication and mention of the grant of the patent: **15.04.2026**
(21) Application number: 24739283.0
(22) Date of filing: 24.06.2024
(51) Int. Cl.: G06T 7/12, A61N 1/36, A61N 1/372, A61N 5/10

(54) **USER TOOLS FOR TREATMENT PLANNING FOR TUMOR TREATING FIELDS**
BENUTZERWERKZEUGE ZUR BEHANDLUNGSPLANUNG FÜR TUMORBEHANDLUNGSFELDER
OUTILS UTILISATEUR POUR LA PLANIFICATION DE TRAITEMENT POUR DES CHAMPS DE TRAITEMENT DE TUMEUR

(30) Priority: 30.06.2023 US 202363524387 P; 21.06.2024 US 202418750190
(43) Date of publication of application: 25.06.2025
(73) Proprietor: Novocure GmbH, 6340 Baar (CH)
(72) Inventor: HERSHKOVICH, Hadas Sara, 31905 Haifa (IL); BAKALO, Oren Ben Zion, 31905 Haifa (IL); SHAMIR, Reuven Ruby, 31905 Haifa (IL); ZIGELMAN, Gil, 31905 Haifa (IL); VARDI, Mor, 31905 Haifa (IL); STEPOVOY, Kirill, 31905 Haifa (IL)
(74) Representative: Boden, Keith McMurray
(86) International application number: PCT/IB2024/056136
(87) International publication number: WO 2025/003865

(56) References cited:
- WO-A1-2021/111186
- US-A1- 2018 160 933
- US-A1- 2020 372 705
- WENGER CORNELIA ET AL: "A Review on Tumor-Treating Fields (TTFields): Clinical Implications Inferred From Computational Modeling", IEEE REVIEWS IN BIOMEDICAL ENGINEERING, vol. 11, 13 February 2018 (2018-02-13), pages 195 - 207, XP011687580, ISSN: 1937-3333, [retrieved on 20180725], DOI: 10.1109/RBME.2017.2765282
- JOSHUA J TIMMONS ET AL: "End-to-end workflow for finite element analysis of tumor treating fields in glioblastomas", PHYSICS IN MEDICINE AND BIOLOGY, INSTITUTE OF PHYSICS PUBLISHING, BRISTOL GB, vol. 62, no. 21, 11 October 2017 (2017-10-11), pages 8264 - 8282, XP020321198, ISSN: 0031-9155, [retrieved on 20171011], DOI: 10.1088/1361-6560/AA87F3
- "Brain and Human Body Modeling : Computational Human Modeling at EMBC 2018", 2019, SPRINGER INTERNATIONAL PUBLISHING, Cham, ISBN: 978-3-030-21293-3, article URMAN NOA ET AL: "Investigating the Connection Between Tumor-Treating Fields Distribution in the Brain and Glioblastoma Patient Outcomes. A Simulation-Based Study Utilizing a Novel Model Creation Technique : Computational Human Modeling at EMBC 2018", pages: 139 - 154, XP055909824, DOI: 10.1007/978-3-030-21293-3_7

## Description

The present invention relates to a computer-implemented method and apparatus for generating at least one transducer layout for delivering tumor treating fields to a subject.

### BACKGROUND

Tumor treating fields (TTFields) are low intensity alternating electric fields within the intermediate frequency range (for example, 50 kHz to 1 MHz), which may be used to treat tumors as described in U.S. Patent No. 7,565,205. TTFields are induced non-invasively into the region of interest by transducers placed on the patient's body and applying AC voltages between the transducers. Conventionally, a first pair of transducers and a second pair of transducers are placed on the subject's body. AC voltage is applied between the first pair of transducers for a first interval of time to generate an electric field with field lines generally running in the front-back direction. Then, AC voltage is applied at the same frequency between the second pair of transducers for a second interval of time to generate an electric field with field lines generally running in the right-left direction. The system then repeats this two-step sequence throughout the treatment.

US-A-2020/0372705 discloses methods, systems and apparatuses for managing placement of transducer arrays on a subject.

US-A-2018/0160933 discloses a method for optimizing an electrode placement layout that maximizes field strength in abnormal tissue using a model of electrical properties based on deformed and modified model templates.

WO-A-2021/111186 discloses a method and apparatus for optimizing transducer array placement based on dose metrics and one or more sets of pairs of positions that satisfy an angular restriction.

Wenger et al (A Review on Tumor-Treating Fields (TTFields): Clinical Implications Inferred From Computational Modeling. IEEE Rev Biomed Eng. 11(2018):195-207) discloses studies of the macroscopic spatial distribution of TTFields generated in the human head using computational modeling.

Timmons et al (End-to-end workflow for finite element analysis of tumor treating fields in glioblastomas. Phys Med Biol. 62(2017):8264-8282) discloses an end-to-end, semi-automatic segmentation-based workflow procedure for generating personalized finite element models for the delineation of intracranial TTFields using real and individual patient MRI datasets.

Urman et al (Investigating the Connection Between Tumor-Treating Fields Distribution in the Brain and Glioblastoma Patient Outcomes. A Simulation-Based Study Utilizing a Novel Model Creation Technique. Brain and Human Body Modeling: Computational Human Modeling at EMBC 2018 [Internet]. Cham (CH): Springer; 2019. Chapter 7) discloses a semi-automatic method for creating realistic head models from glioblastoma patient MRI using a deformable template and atlas-based registration.

### SUMMARY OF THE INVENTION

In one aspect the present invention provides a computer-implemented method for generating at least one transducer layout for delivering tumor treating fields to a subject according to claim 1.

In another aspect the present invention provides an apparatus for generating at least one transducer layout for delivering tumor treating fields to a subject according to claim 9.

Embodiments of the invention are disclosed in the dependent claims.

### BRIEF DESCRIPTION OF THE DRAWINGS

FIG. 1 is a flowchart depicting a method for generating at least one transducer layout for delivering TTFields to a subject according to one or more embodiments described herein.
FIGS. 2-4 are example user interfaces of a computer-based application for generating at least one transducer layout for delivering TTFields to a subject.
FIG. 5 is a flowchart depicting a method for generating at least one transducer layout for delivering TTFields to a subject according to one or more embodiments described herein.
FIG. 6 is an example user interface of a computer-based application for generating at least one transducer layout for delivering TTFields to a subject.
FIG. 7 is a flowchart depicting a method for generating at least one transducer layout for delivering TTFields to a subject according to one or more embodiments described herein.
FIG. 8 is an example user interface of a computer-based application for generating at least one transducer layout for delivering TTFields to a subject.
FIG. 9 is a flowchart depicting a method for generating at least one transducer layout for delivering TTFields to a subject according to one or more embodiments described herein.
FIG. 10 is an example user interface of a computer-based application for generating at least one transducer layout for delivering TTFields to a subject.
FIG. 11 is a flowchart depicting a method for generating at least one transducer layout for delivering TTFields to a subject according to one or more embodiments described herein.
FIG. 12 depicts an example system for delivering TTFields to a subject's body according to one or more embodiments described herein.
FIG. 13 depicts an example placement of transducers on a subject's head according to one or more embodiments described herein.
FIG. 14 depicts an example apparatus for performing the disclosed methods according to one or more embodiments described herein.

### DESCRIPTION OF EMBODIMENTS

This application describes exemplary user tools for treatment planning for administering TTFields to a subject.

In general, one or more pairs of transducers are positioned on the subject's body and used to alternately apply TTFields to the subject's body. Generally, it is preferred that there are at least two pairs of transducers that are arranged to target a specific location or structure (e.g., a tumor) within the subject. Accordingly, proper placement of transducers is useful for treating the subject. Conventional treatment planning uses a series of measurements taken from a subject's magnetic resonance imaging (MRI) scans to measure aspects of the subject (e.g., the subject's head size, a location of the tumor, a size of the tumor, and/or the like including combinations and/or multiples thereof). Using these measurements, a layout for the transducers is generated.

The inventors have now recognized that a need exists for treatment planning capable of providing more customized layouts for applying TTfields to a subject.

Embodiments described herein provide for planning for TTFields based on medical images, such as MRI medical images and/or computed tomography (CT) medical images. The planning uses the MRI medical images and/or the CT medical images to generate one or more transducer layouts for application of TTFields to a subject. As will be evident by this disclosure, one or more techniques are provided for generating the transducer layout(s). For example, an embodiment for generating a transducer layout uses an auto matching tool for automatically registering MRI medical images and CT medical images. Another embodiment for generating a transducer layout uses an overlap segmenting tool that automatically segments slices in between two user-segmented slices. That is, a user manually segments two slices, and the overlap segmenting tool automatically segments slices in between the two user-segmented slices. Another embodiment for generating a transducer layout uses a split segmenting tool for splitting a segmented area. For example, after a user manually segments a slice, the split segmenting tool splits the segmented area based on a gray scale value of pixel in the medical images, such as using a gray scale slider. Another embodiment for generating a transducer layout uses a clean-up segmenting tool for improving a segmented area. For example, after a slice is manually segmented, the clean-up segmenting tool can be used to smooth edges of the segmented area, to remove oddly segmented islands and/or holes, and/or the like including combinations and/or multiples thereof. Another embodiment for generating a transducer layout uses an avoidance area tool to identify areas on the subject that should be avoided for placing the transducer array. It should be appreciated that the auto matching tool, the overlap segmenting tool, the split segmenting tool, the clean-up segmenting tool, and/or the avoidance area tool can be used individually and/or in different combinations and can be employed at different stages of the process for generating transducer layouts. Further, one or more of the tools can be combined, such as the split segmenting tool and the clean-up segmenting tool.

The embodiments described herein further provide a practical application to generating transducer layouts based on medical images for the user. By using the medical images, such as MRI medical images and CT medical images, the subject's tissue conductivity is considered when generating transducer layouts for treating the subject. By using tools (e.g., the auto matching tool, the overlap segmenting tool, the split segmenting tool, the clean-up segmenting tool, and/or the avoidance area tool), the medical images can be used more effectively to generate transducer layouts for application of TTFields to the subject. For example, the tools aid in pinpointing the area of the subject to be treated (e.g., where to focus an electric field relative to the subject). These and other technical improvements may be realized using the one or more embodiments described herein.

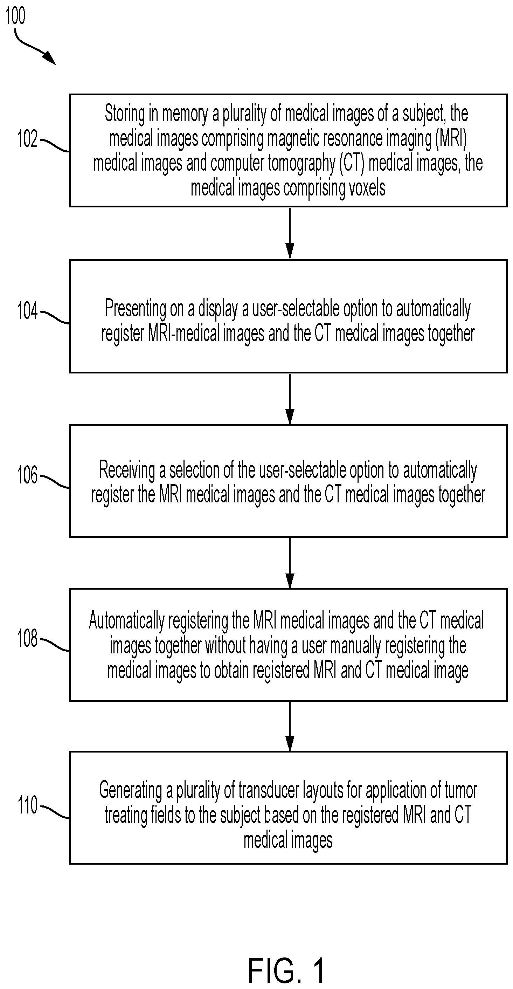

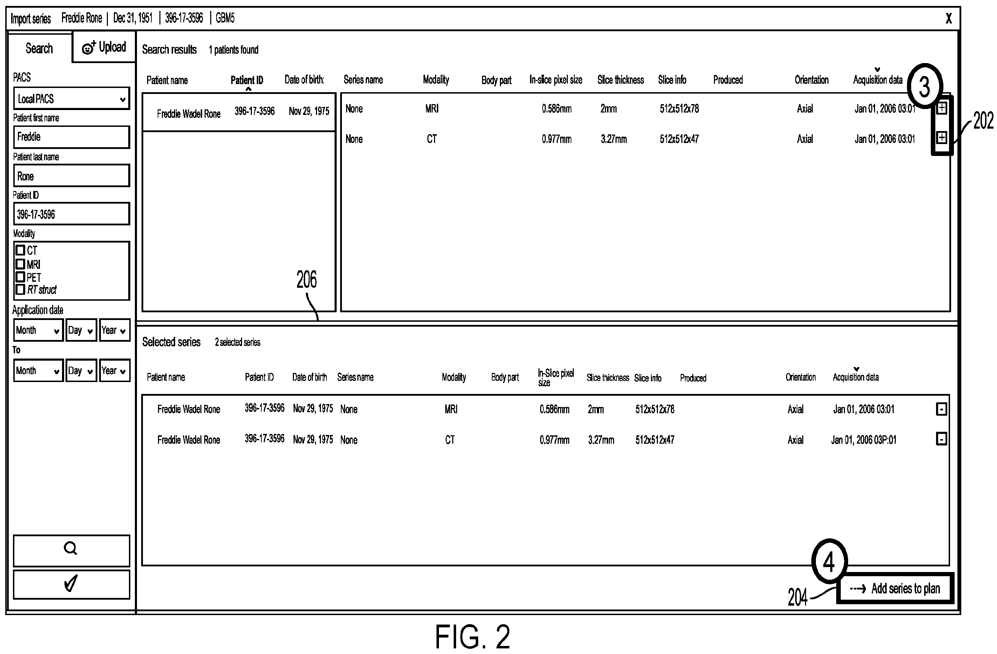

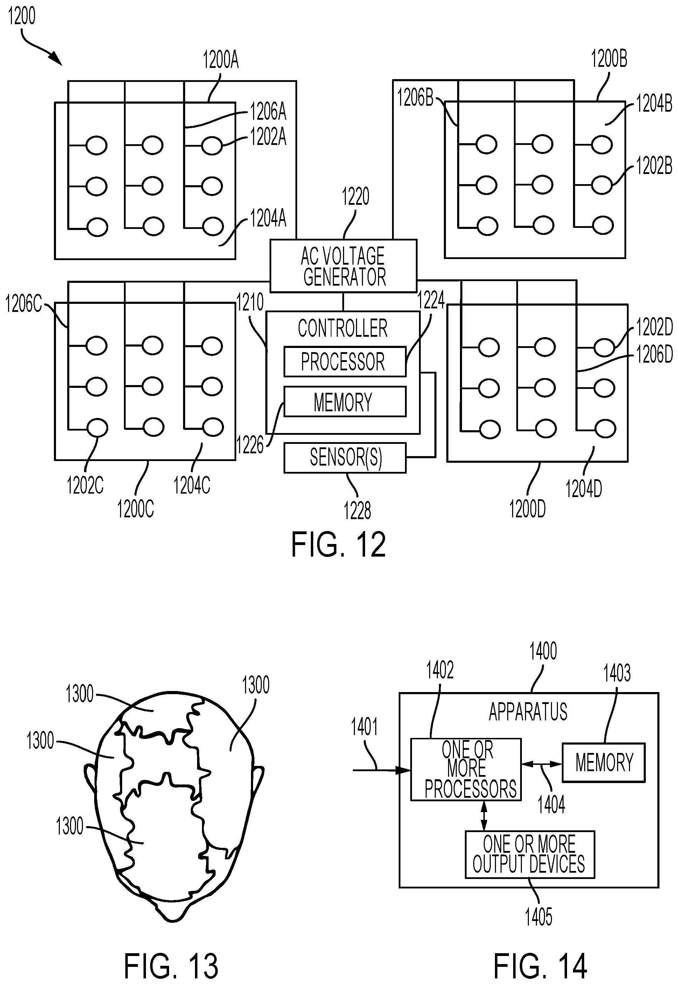

FIG. 1 is a flowchart depicting a method 100 for generating at least one transducer layout for delivering TTFields to a subject. In this example, the method 100 automatically registers MRI medical images and CT medical images. The method 100 can be implemented by any suitable system or apparatus, such the system of FIG 12 and/or the apparatus of FIG. 14. The method 100 is now described with reference to the example user interfaces of a computer-based application for generating at least one transducer layout for delivering TTFields to a subject as shown in FIGS. 2-4; however, the method 100 is not so limited. While an order of operations is indicated in FIG. 1 for illustrative purposes, the timing and ordering of such operations may vary where appropriate without negating the purpose and advantages of the examples set forth in detail throughout the remainder of this disclosure.

At block 102, the method 100 stores in a memory (e.g., the memory 1226 of FIG. 12, the memory 1403 of FIG. 14, and/or the like including combinations and/or multiples thereof) a plurality of medical images of a subject. The medical images may include, for example, MRI medical images and CT medical images, and the medical images include voxels. For example, FIG. 2 shows a user interface of a computer-based application for generating transducer layouts. In FIG. 2, the user interface is provided for loading medical images in memory, such as the MRI medical images and the CT medical images. A user can load medical images by selecting the + buttons 202 and then selecting the add series to plan button 204 to add the medical images. According to an embodiment, the user-selectable listing of MRI medical images and the user-selectable listing of CT medical images are presented concurrently on the display, for example as the selected series 206.

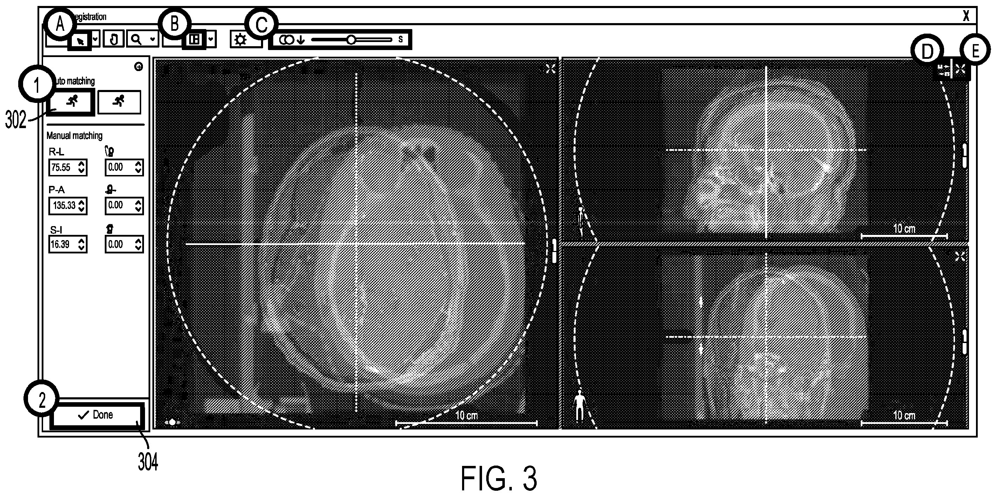

At block 104, the method 100 presents on a display a user-selectable option to automatically register the MRI medical images and the CT medical images together. For example, FIG. 3 shows a user interface for performing automatically registering (e.g., auto matching), such as by an auto matching tool. Auto matching involves automatically registering MRI and CT medical images together.

At block 106, the method 100 receives a selection of the user-selectable option to automatically register the MRI medical images and the CT medical images together. Referring to FIG. 3, the user initiates the automatic registering by selecting the auto matching button 302 and then, after making any additional selections, selecting the done button 304, at which point auto matching is performed.

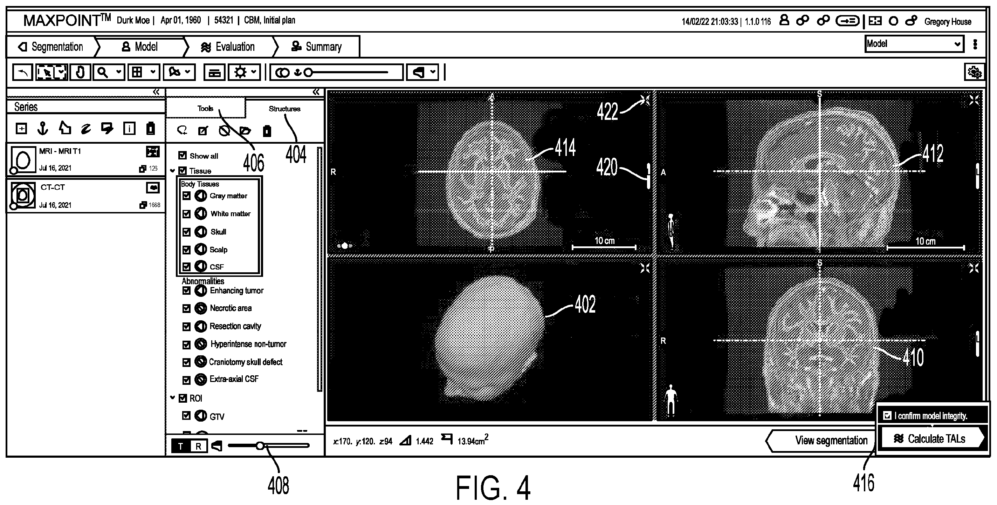

At block 108, the method 100 automatically registers the MRI medical images and the CT medical images together without having a user manually registering the medical images to obtain registered MRI and CT medical image. According to an embodiment, when the MRI medical images and the CT medical images are automatically registered together, the MRI medical images and the CT medical images are aligned and linked together. According to an embodiment, when the MRI medical images and the CT medical images are automatically registered together, the CT medical images are registered with respect to the MRI medical images. However, in some cases, the user may also manually register some or all the MRI and CT medical images. For example, the method 100 may present on the display one or more user-selectable icons to manually register the MRI medical images and the CT medical images together to obtain registered MRI and CT medical images. Registering the CT medical images to the MRI medical images may provide improved bone definition that improves the accuracy of a three-dimensional (3D) model of the subject. For example, FIG. 4 depicts an interface that shows a 3D rendering 402 of the subject. That is, the 3D rendering 402 depicts a rendering of an outer surface of a patient's head. In some examples, the 3D rendering 402 can depict transducer arrays placed on the outer surface of the patient's head. Normal structures appear on the structures tab 404 and can be modified using the tools tab 406.

According to one or more embodiments described herein, the method 100 presents on the display a user-selectable icon to selectively change an opacity between corresponding slices through the MRI medical images and CT images, where the corresponding slices through the MRI medical images and CT images are overlaid on each other. For example, FIG. 4 shows an opacity slider 408 for selectively changing the opacity between corresponding slices through the MRI and CT images. According to an embodiment, the slider 408 can have a first end and a second end such that when the first end of the slider icon is selected, only the corresponding slices through the MRI medical images are displayed, and such that when the second end of the slider icon is selected, only the corresponding slices through the CT images are displayed.

As shown in FIG. 4, the method 100 can present on the display a user-selectable icon to display simultaneously a front view 410, a side view 412, and a top view 414 of corresponding slices through the MRI medical images and CT images, where the corresponding slices through the MRI medical images and CT images are overlaid on each other as shown. The front view 410, the side view 412, and/or the top view 414 can be manipulated by a user. For example, the method 100 can presents on the display one or more user-selectable icons 420 to selectively zoom-in or zoom-out of one or more of the front view 410, the side view 412, and/or the top view 414 of corresponding slices through the MRI medical images and CT images, where the corresponding slices through the MRI medical images and CT images are overlaid on each other. The method 100 can further present on the display one or more user-selectable icons 422 to selectively display one of the front view 410, the side view 412, and/or the top view 414 of corresponding slices through the MRI medical images and CT images, where the corresponding slices through the MRI medical images and CT images are overlaid on each other.

In some examples, the slices can be displayed before and after registration. For example, the method 100 can display on the display corresponding slices through the MRI medical images and CT images prior to registering and can display on the display corresponding slices through the registered MRI medical images and CT images.

At block 110, the method 100 generates a plurality of transducer layouts for application of tumor treating fields to the subject based on the registered MRI and CT medical images. For example, with reference to FIG. 4, after the registration is performed, the user can select the calculate transducer array layouts (TALs) button 416 to calculate a transducer array layout or multiple transducer array layouts.

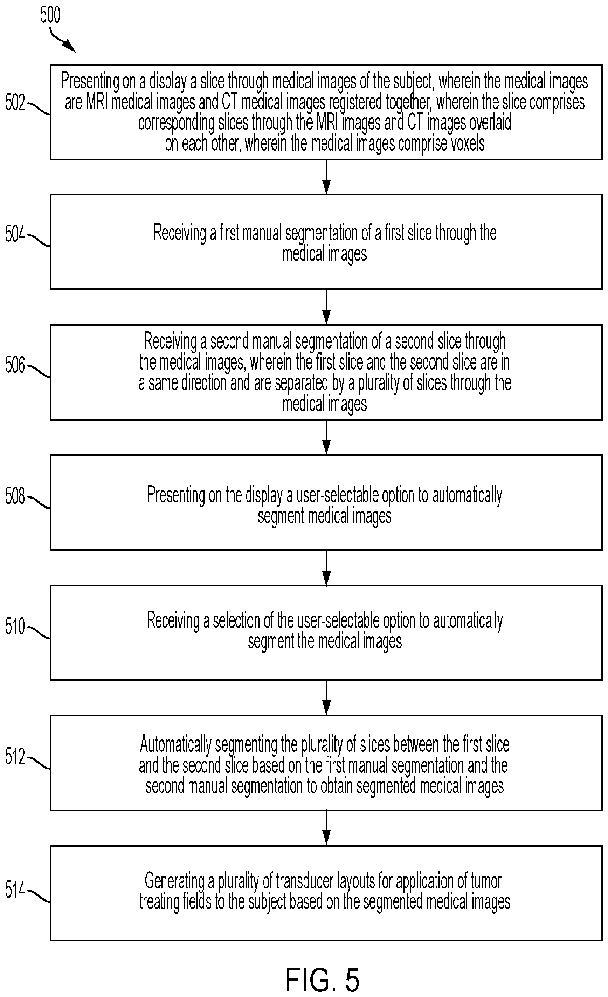

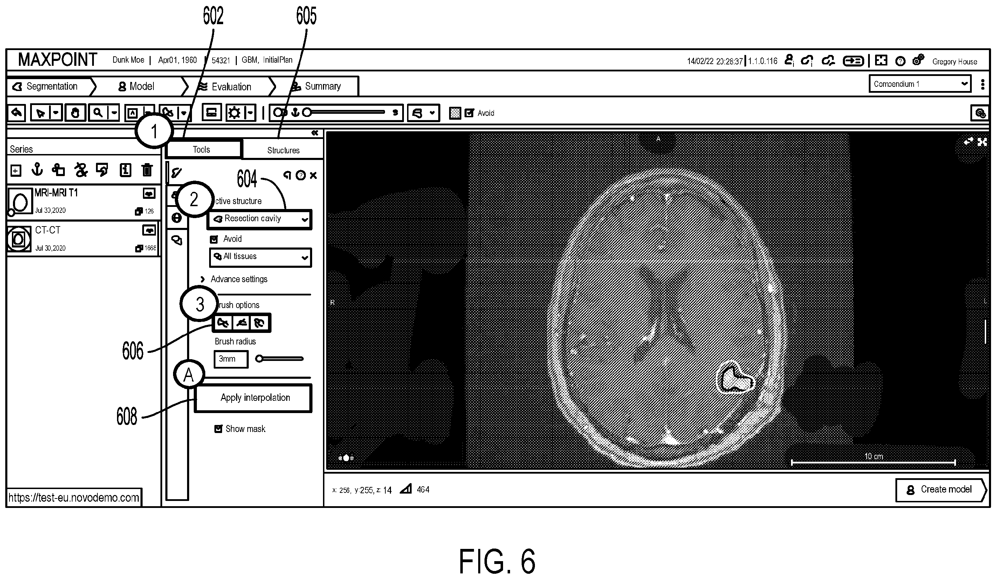

FIG. 5 is a flowchart depicting a method 500 for generating at least one transducer layout for delivering TTFields to a subject. In this example, the method 500 automatically performs segmentation of slices. The method 500 can be implemented by any suitable system or apparatus, such the system of FIG. 12 and/or the apparatus of FIG. 14. The method 500 is now described with reference to the example user interface of a computer-based application for generating at least one transducer layout for delivering TTFields to a subject as shown in FIG. 6; however, the method 500 is not so limited. While an order of operations is indicated in FIG. 5 for illustrative purposes, the timing and ordering of such operations may vary where appropriate without negating the purpose and advantages of the examples set forth in detail throughout the remainder of this disclosure.

At block 502, the method 500 presents on a display a slice through medical images of the subject. The medical images can include MRI medical images and CT medical images registered together, such as using the method 100. The slice includes corresponding slices through the MRI medical images and CT images overlaid on each other, and the medical images include voxels.

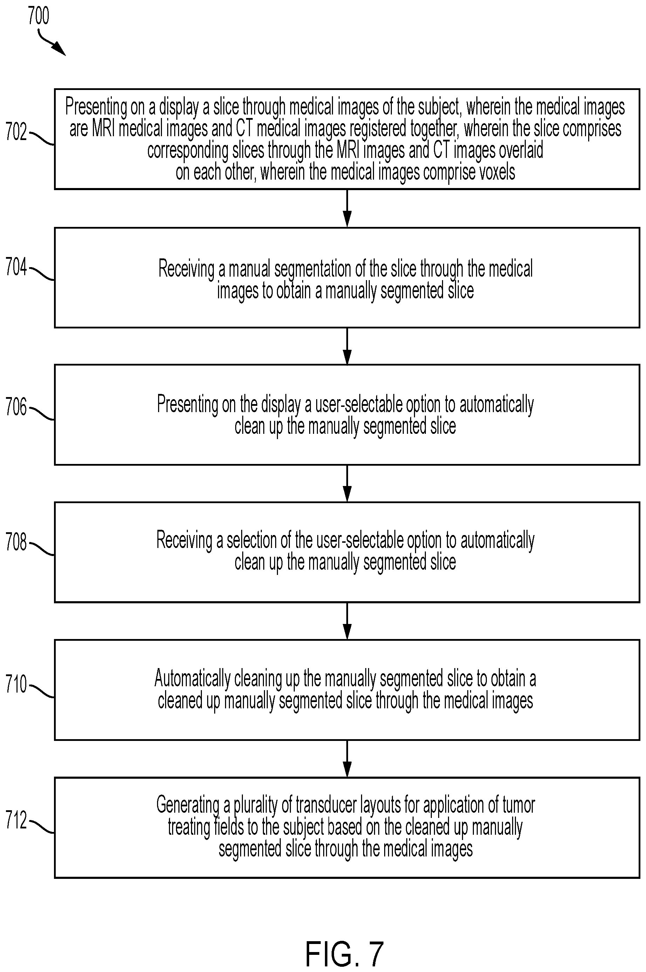

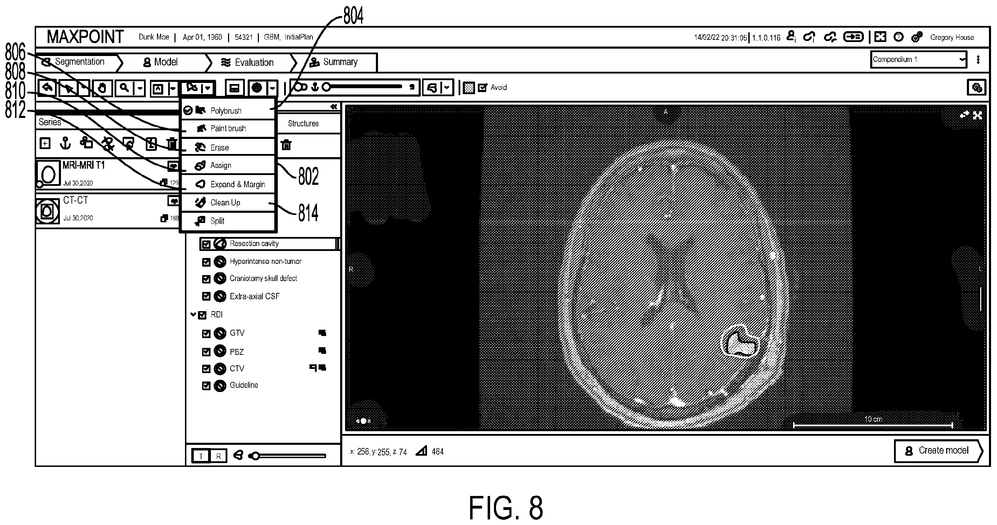

At block 504, the method 500 receives a first manual segmentation of a first slice through the medical images. For example, the user selects a first slice on the medical images. For example, FIG. 6 shows a user interface for automatically segmenting the medical images. In this example, the user can select a tools tab 602 to begin contouring structures. The user can then select an active structure via dropdown 604 to identify a type of structure to contour (e.g., a resection cavity). As an option, the user may also select an active structure for segmenting by selecting the structures tab 605. In the tools tab 602, the user can then select a brush 606 (or any other suitable tool) to perform the segmenting. Manual segmentation is discuss further below with respect to FIGS. 7 and 8.

At block 506, the method 500 receives a second manual segmentation of a second slice through the medical images. For example, the user selects a second slice on the medical images. The first slice and the second slice may be in a same direction and may be separated by a plurality of slices through the medical images.

At block 508, the method 500 presents on the display a user-selectable option to automatically segment medical images. According to one or more embodiments described herein, referring to FIG. 6, an interpolation tool 608 can be used to expedite the segmenting. For example, a structure can be segmented in a first slice (block 504), one or more subsequent slices can be skipped, and then the structure can be segmented again on a next slice following the skipped slices (block 506).

At block 510, the method 500 receives a selection of the user-selectable option to automatically segment the medical images. For example, with reference to FIG. 6, the interpolation tool 608 can be selected by the user to apply the segmenting for the skipped slices, where the interpolation segmenting is performed based on the segmenting performed on the slices adjacent to the skipped slices. That is, the user can select the interpolation tool to initiate the automatic segmentation.

At block 512, the method 500 automatically segments the plurality of slices between the first slice and the second slice based on the first manual segmentation and the second manual segmentation to obtain segmented medical images. The automatically segmented slices can be automatically segmented by interpolating between manually segmented slices through the medical images. The automatically segmented slices through the medical images are situated between manually segmented slices (e.g., the first slice and the second slice) through the medical images, where the automatically segmented slices may be in a same direction as the manually segmented slices, where the automatically segmented slices may be automatically segmented based on the manually segmented slices. As an example, a number of automatically segmented slices between a pair of manually segmented slices is between 2 and 20. As another example, a number of automatically segmented slices between a pair of manually segmented slices is between 2 and 5. Other numbers of automatically segmented slices are also possible.

In some embodiments, prior to automatically segmenting, any manually segmented slices may be cleaned up using, for example, the method described below with respect to FIG. 7 for cleaning up slices. In some embodiments, after automatically segmenting, the segmented slices may be cleaned up using, for example, the method described below with respect to FIG. 7 for cleaning up slices.

At block 514, the method 500 generates a plurality of transducer layouts for application of tumor treating fields to the subject based on the segmented medical images. For example, with reference to FIG. 4, after the automatic segmentation is performed, the user can select the calculate TALs button 416 to calculate a transducer array layout or multiple transducer array layouts.

The method 500 also supports manual segmentation in one or more embodiments, such as in blocks 504 and 506. For example, the method 500 can present on the display one or more user-selectable icons to manually segment slices through the medical images to obtain manually segmented slices through the medical images. The plurality of transducer layouts for application of tumor treating fields to the subject can be further based on the manually segmented slices through the medical images.

FIG. 7 is a flowchart depicting a method 700 for generating at least one transducer layout for delivering TTFields to a subject. In this example, the method 700 splits and cleans up segments. The method 700 can be implemented by any suitable system or apparatus, such the system of FIG. 12 and/or the apparatus of FIG. 14. The method 700 is now described with reference to the example user interface of a computer-based application for generating at least one transducer layout for delivering TTFields to a subject as shown in FIG. 8; however, the method 700 is not so limited. While an order of operations is indicated in FIG. 7 for illustrative purposes, the timing and ordering of such operations may vary where appropriate without negating the purpose and advantages of the examples set forth in detail throughout the remainder of this disclosure.

At block 702, the method 700 presents on a display a slice through medical images of the subject. The medical images are MRI medical images and CT medical images registered together. The slice includes corresponding slices through the MRI medical images and CT images overlaid on each other, and the medical images include voxels.

At block 704, the method 700 receives a manual segmentation of the slice through the medical images to obtain a manually segmented slice. For example, the user interface of FIG. 8 shows multiple user-selectable options 802 (e.g., user-selectable icons) to manually segment the slice, such as a user-selectable icon 804 to autofill a region (e.g., a polybrush option), a user-selectable icon 806 to select the region without autofill (e.g., a paint brush option), a user-selectable icon 808 to erase a segmentation (e.g., an erase option), a user-selectable icon 810 to assign a tissue type (e.g., an assign option), a user-selectable icon 812 to expand a border of the region (e.g., an expand & margin option), and/or the like including combinations and/or multiples thereof.

At block 706, the method 700 presents on the display a user-selectable option to automatically clean up the manually segmented slice. For example, the user-selectable options 802 of FIG. 8 can include a user-selectable icon 814 to automatically clean up a slice.

At block 708, the method 700 receives a selection of the user-selectable icon 814 to automatically clean up the manually segmented slice.

At block 710, the method 700 automatically cleans up the manually segmented slice to obtain a cleaned up manually segmented slice through the medical images. For example, the manually segmented slice is automatically cleaned up by splitting a segmented area of the manually segmented slice into a first portion and a second portion based on gray scale values. The first portion is identified as normal tissue and the second portion is identified as abnormal tissue. The abnormal tissue can include, for example, a tumor, necrotic tissue, a prior surgical area, and/or the like including combinations and/or multiples thereof. As an example, the segmented area is split based on a threshold adjusted by a user-selectable slider of gray scale values (e.g., a gray scale slider). In some embodiments, a user-adjustable icon (e.g., a slider, a knob, or a field with menu-selectable values for a gray scale value) may be presented on the display to identify a threshold for the gray scale values, where gray scale values on a first side of the threshold are designated as being in the first portion, and where gray scale values on a second side of the threshold are designated as being in the second portion. According to one or more embodiments described herein, the manually segmented slice can be automatically cleaned up by, for example, smoothing one or more edges of a segmented area of the manually segmented slice, removing one or more discontinuous segmented areas outside a larger segmented area of the manually segmented slice, removing one or more non-segmented areas inside a segmented area of the manually segmented slice, and/or the like including combinations and/or multiples thereof.

At block 712, the method 700 generates a plurality of transducer layouts for application of tumor treating fields to the subject based on the cleaned up manually segmented slice through the medical images. For example, with reference to FIG. 4, after the clean-up is performed, the user can select the calculate transducer array layouts (TALs) button 416 to calculate a transducer array layout or multiple transducer array layouts.

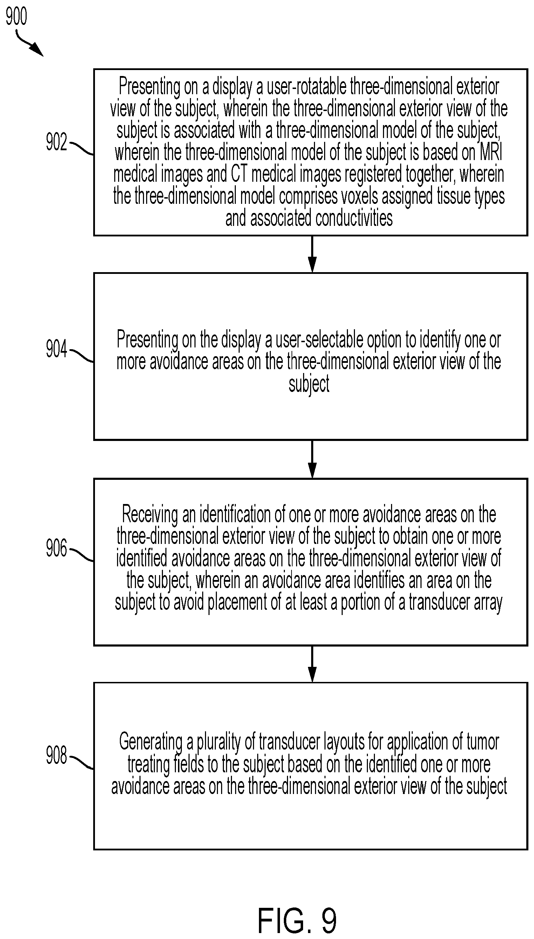

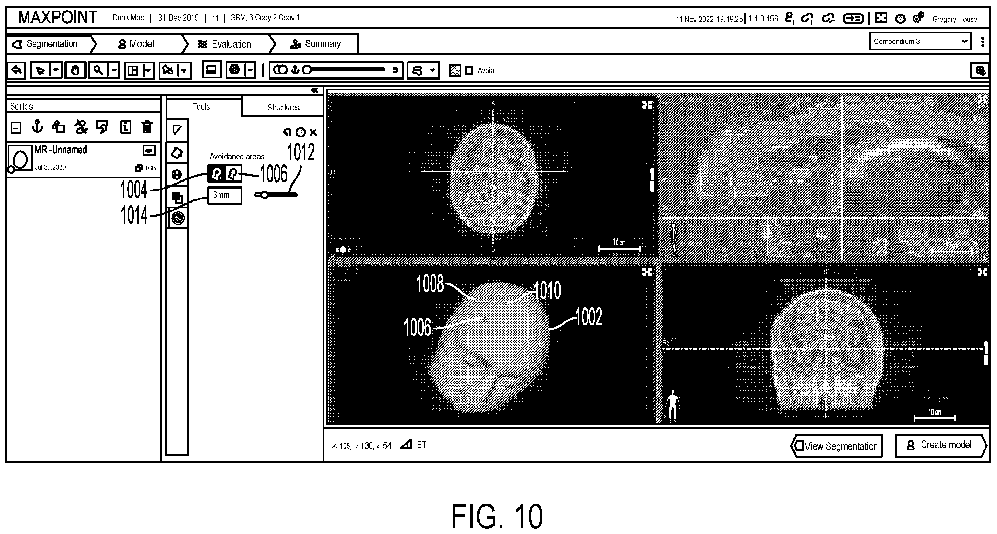

FIG. 9 is a flowchart depicting a method 900 for generating at least one transducer layout for delivering TTFields to a subject. In this example, the method 900 identifies areas to avoid placing transducer arrays. The method 900 can be implemented by any suitable system or apparatus, such the system of FIG. 12 and/or the apparatus of FIG. 14. The method 900 is now described with reference to the example user interface of a computer-based application for generating at least one transducer layout for delivering TTFields to a subject as shown in FIG. 10; however, the method 900 is not so limited. While an order of operations is indicated in FIG. 9 for illustrative purposes, the timing and ordering of such operations may vary where appropriate without negating the purpose and advantages of the examples set forth in detail throughout the remainder of this disclosure.

At block 902, the method 900 presents on a display a user-rotatable three-dimensional exterior view of the subject. The three-dimensional exterior view of the subject is associated with a 3D model of the subject. The 3D model of the subject may be based on MRI medical images and CT medical images registered together, as described herein, or only on MRI medical images, or only CT medical images. The 3D model includes voxels assigned tissue types and associated conductivities. FIG. 10 shows a user interface displaying several views, both interior views and an exterior view as a 3D rendering 1002, of a subject. According to one or more embodiments described herein, a three-dimensional conductivity map is part of the 3D model. The three-dimensional conductivity map may depict the electrical conductivity of the body tissues of the subject.

At block 904, the method 900 presents on the display a user-selectable option to identify one more avoidance areas on the three-dimensional exterior view of the subject. An avoidance area may identify an area on the subject to avoid placement of at least a portion of a transducer array. For example, in FIG. 10, an avoidance area tool 1004 can be used to identify one more avoidance areas on the three-dimensional exterior view (e.g., the 3D rendering 1002) of the subject.

At block 906, the method 900 receives an identification of one or more avoidance areas on the three-dimensional exterior view of the subject to obtain one or more identified avoidance areas on the three-dimensional exterior view of the subject. According to one or more embodiments described herein, the method 900 presents on the display at least one identified avoidance area on the three-dimensional exterior view of the subject. For example, in FIG. 10, once the avoidance area tool 1004 is selected, the cursor can be moved by the user, and an area of the 3D rendering 1002 of the subject can be selected by the user to locate an avoidance area. One or more areas of the 3D rendering 1002 of the subject can be selected as avoidance areas. The 3D rendering 1002 of the subject can be rotated and/or scaled for ease in selecting one or more avoidance areas. To remove an avoidance area, a remove avoidance area tool 1006 may be selected, and the cursor can be moved by the user to a previously identified avoidance area to be identified and removed from the 3D rendering 1002. For example, in FIG. 10, three avoidance areas 1006, 1008, and 1010 are shown as the three substantially circular areas on the 3D rendering 1002 of the subject. According to one or more embodiments described herein, the method 900 presents on the display at least one of a user-selectable icon 1012 to adjust a size, as shown in window 1014, of an avoidance area or a user-selectable icon to select a shape of an avoidance area. For example, the size of the avoidance area is adjustable between approximately 1 mm to approximately 15 mm, although other sizes are possible. As an example, the shape of the avoidance area is approximately circular, although other shapes are also possible. According to one or more embodiments described herein, the method 900 can present on the display a user-selectable icon to receive a shape of at least one avoidance area manually drawn by a user.

At block 908, the method 900 generates a plurality of transducer layouts for application of tumor treating fields to the subject based on the identified one or more avoidance areas on the three-dimensional exterior view of the subject. For example, with reference to FIG. 4, after the automatic segmentation is performed, the user can select the calculate TALs button 416 to calculate a transducer array layout or multiple transducer array layouts. According to one or more embodiments described herein, the method 900 further presents on the display a first dosage of tumor treating fields delivered to the subject for a first transducer layout of the plurality of transducer layouts such that the first transducer layout avoids all of the identified avoidance areas. According to one or more embodiments described herein, the method 900 further presents on the display a second dosage of tumor treating fields delivered to the subject for a second transducer layout of the plurality of transducer layouts such that the second transducer layout is located on at least one of the identified avoidance areas.

The transducer layouts can take different forms in various embodiments. For example, a first transducer layout of the plurality of transducer layouts avoids the identified avoidance areas. In this example, the first transducer layout includes one or more electrode elements arranged such that none of the electrode elements are located on any of the identified avoidance areas. As another example, a first transducer layout of the plurality of transducer layouts avoids the identified avoidance areas. In this example, the first transducer layout includes an adhesive portion for attaching the first transducer layout to the subject arranged such that none of the adhesive portion is located on any of the identified avoidance areas.

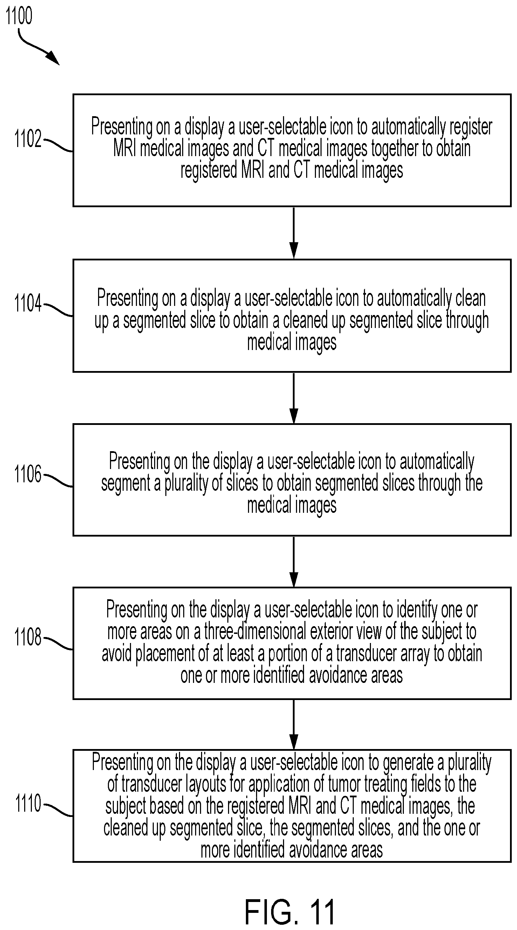

FIG. 11 is a flowchart depicting a method 1100 for generating at least one transducer layout for delivering TTFields to a subject. The method 1100 can be implemented by any suitable system or apparatus, such the system of FIG. 12 and/or the apparatus of FIG. 14. While an order of operations is indicated in FIG. 11 for illustrative purposes, the timing and ordering of such operations may vary where appropriate without negating the purpose and advantages of the examples set forth in detail throughout the remainder of this disclosure.

At block 1102, the method 1100 presents on a display a user-selectable icon to automatically register MRI medical images and CT medical images together to obtain registered MRI and CT medical images. As an example, the user-selectable auto matching button 302 in the user interface of FIG. 3 is user selectable to initiate automatic registering. FIG. 1 shows a method 100 that automatically registers MRI medical images and CT medical images as described herein.

At block 1104, the method 1100 presents on the display a user-selectable icon to automatically clean up a segmented slice to obtain a cleaned up segmented slice through medical images. As an example, the user-selectable icon 814 in the user interface of FIG. 8 is user selectable to initiate automatic clean up of a slice through medical images. FIG. 7 shows a method 700 that splits and cleans up segments as described herein. As an example, a slice may be automatically cleaned up after manual segmentation or after automatic segmentation in block 1106.

At block 1106, the method 1100 presents on the display a user-selectable icon to automatically segment a plurality of slices to obtain segmented slices through the medical images. As an example, the interpolation tool 608 in the user interface of FIG. 6 is user selectable to initiate automatic segmentation of slices. FIG. 5 shows a method 500 that automatically performs segmentation of slices as described herein.

At block 1108, the method 1100 presents on the display a user-selectable icon to identify one or more areas on a three-dimensional exterior view of the subject to avoid placement of at least a portion of a transducer array to obtain one or more identified avoidance areas. As an example, the avoidance area tool 1004 in the user interface of FIG. 10 is user selectable to identify one more avoidance areas on the three-dimensional exterior view (e.g., the 3D rendering 1002) of the subject. FIG. 9 shows a method 900 that identifies areas to avoid placing transducer arrays as described herein.

At block 1110, the method 1100 presents on the display a user-selectable icon to generate a plurality of transducer layouts for application of tumor treating fields to the subject based on the registered MRI and CT medical images, the cleaned up segmented slice, the segmented slices, and the one or more identified avoidance areas. As an example, the calculate TALS button 416 in FIG. 4 is user selectable to generate a plurality of transducer layouts for application of tumor treating fields to the subject based on the registered MRI and CT medical images, the cleaned up segmented slice, the segmented slices, and the one or more identified avoidance areas.

FIG. 12 depicts an example apparatus 1200 to apply alternating electric fields (e.g., TTFields) to the subject's body. The system may be used for treating a target region of a subject's body with an alternating electric field. In an example, the target region may be in the subject's brain, and an alternating electric field may be delivered to the subject's body via two pairs of transducer arrays positioned on a head of the subject's body (such as, for example, in FIG. 13, which has four transducers 1300). In another example, the target region may be in the subject's torso, and an alternating electric field may be delivered to the subject's body via two pairs of transducer arrays positioned on at least one of a thorax, an abdomen, or one or both thighs of the subject's body. Other transducer array placements on the subject's body may be possible.

The example apparatus 1200 depicts an example system having four transducers (or "transducer arrays") 1200A-D. Each transducer 1200A-D may include substantially flat electrode elements 1202A-D positioned on a substrate 1204A-D and electrically and physically connected (e.g., through conductive wiring 1206A-D). The substrates 1204A-D may include, for example, cloth, foam, flexible plastic, and/or conductive medical gel. Two transducers (e.g., 1200A and 1200D) may be a first pair of transducers configured to apply an alternating electric field to a target region of the subject's body. The other two transducers (e.g., 1200B and 1200C) may be a second pair of transducers configured to similarly apply an alternating electric field to the target region.

The transducers 1200A-D may be coupled to an AC voltage generator 1220, and the system may further include a controller 1210 communicatively coupled to the AC voltage generator 1220. The controller 1210 may include a computer having one or more processors 1224 and memory 1226 accessible by the one or more processors. The memory 1226 may store instructions that when executed by the one or more processors control the AC voltage generator 1220 to induce alternating electric fields between pairs of the transducers 1200A-D according to one or more voltage waveforms and/or cause the computer to perform one or more methods disclosed herein. The controller 1210 may monitor operations performed by the AC voltage generator 1220 (e.g., via the processor(s) 1224). One or more sensor(s) 1228 may be coupled to the controller 1210 for providing measurement values or other information to the controller.

The electrode elements 1202A-D may be capacitively coupled. In one example, the electrode elements 1202A-D are ceramic electrode elements coupled to each other via conductive wiring 1206A-D. When viewed in a direction perpendicular to its face, the ceramic electrode elements may be circular shaped or non-circular shaped. In other embodiments, the electrode elements are not capacitively coupled, and there is no dielectric material (such as ceramic, or high dielectric polymer layer) associated with the electrode elements.

The structure of the transducers 1200A-D may take many forms. The transducers may be affixed to the subject's body or attached to or incorporated in clothing covering the subject's body. The transducer may include suitable materials for attaching the transducer to the subject's body. For example, the suitable materials may include cloth, foam, flexible plastic, and/or a conductive medical gel. The transducer may be conductive or nonconductive.

The transducer may include any desired number of electrode elements. Various shapes, sizes, and materials may be used for the electrode elements. Any constructions for implementing the transducer (or electric field generating device) for use with embodiments of the invention may be used as long as they are capable of (a) delivering TTFields to the subject's body and (b) being positioned at the locations specified herein. In certain embodiments, at least one electrode element of the first, the second, the third, or the fourth transducer can include at least one ceramic disk that is adapted to generate an alternating electric field. In non-limiting embodiments, at least one electrode element of the first, the second, the third, or the fourth transducer includes a polymer film that is adapted to generate an alternating field.

FIG. 14 depicts an example computer apparatus for use with the embodiments herein. As an example, the apparatus 1400 may be a computer to implement certain inventive techniques disclosed herein, such as generating transducer layouts for delivering TTFields to a subject. For example, the blocks of FIGS. 1, 5, 7, 9, and/or 11 may be performed by a computer, such as the apparatus 1400. As an example, the apparatus 1400 may be a controller apparatus to apply the alternating electric fields (e.g., TTFields) with modulated electric fields for the embodiments herein. The apparatus 1400 may be used as the controller 1210 of FIG. 2. The apparatus 1400 may include one or more processors 1402, memory 1403, one or more input devices, and one or more output devices 1405.

In one example, based on input 1401, the one or more processors 1402 may generate control signals to control the voltage generator to implement an embodiment of the present disclosure. In one example, the input 1401 is user input. In another example, the input 1401 may be from another computer in communication with the apparatus 1400. The input 1401 may be received in conjunction with one or more input devices (not shown) of the apparatus 1400.

The memory 1403 may be accessible by the one or more processors 1402 (e.g., via a link) so that the one or more processors 1402 can read information from and write information to the memory 1403. The memory 1403 may store instructions that when executed by the one or more processors 1402 implement one or more embodiments of the present disclosure.

The one or more output devices 1405 may provide the status of the operation of the invention, such as transducer array selection, voltages being generated, and other operational information. The output device(s) 1405 may provide visualization data according to certain embodiments of the invention.

The apparatus 1400 may be an apparatus for generating at least one transducer layout for delivering tumor treating fields to a subject, the apparatus including: one or more processors (such as one or more processors 1402); and memory (such as memory 1403) accessible by the one or more processors, the memory storing instructions that when executed by the one or more processors, cause the apparatus to perform one or more methods described herein.

The memory 1403 may be a non-transitory processor readable medium containing a set of instructions thereon for generating at least one transducer layout for delivering tumor treating fields to a subject, wherein when executed by a processor (such as processor 1402), the instructions cause the processor to perform one or more methods described herein.

Optionally, for each embodiment described herein, the voltage generation components supply the transducers with an electrical signal having an alternating current waveform at frequencies in a range from about 50 kHz to about 1 MHz and appropriate to deliver TTFields treatment to the subject's body.

Embodiments illustrated under any heading or in any portion of the disclosure may be combined with embodiments illustrated under the same or any other heading or other portion of the disclosure unless otherwise indicated herein or otherwise clearly contradicted by context. For example, and without limitation, embodiments described in dependent claim format for a given embodiment ( e.g., the given embodiment described in independent claim format) may be combined with other embodiments (described in independent claim format or dependent claim format).

Numerous modifications, alterations, and changes to the described embodiments are possible without departing from the scope of appended claims. It is intended that the present invention not be limited to the described embodiments, but that it has the full scope defined by the appended claims.

## Claims

1. A computer-implemented method for generating at least one transducer layout for delivering tumor treating fields to a subject, the method comprising:
presenting on a display one or more user-selectable icons (420, 422) to display a slice through medical images of the subject;
presenting on the display one or more user-selectable icons (802) to manually segment the slice through the medical images to obtain a manually-segmented slice through the medical images;
presenting on the display a user-selectable icon (416) to generate a plurality of transducer layouts for application of tumor treating fields to the subject;
**characterized by**:
the medical images being magnetic resonance imaging (MRI) medical images and computer tomography (CT) medical images registered together, wherein the slice comprises corresponding slices through the MRI medical images and the CT medical images overlaid on each other, and the medical images comprise voxels;
presenting on the display a user-selectable icon (814) to automatically clean up the manually-segmented slice to obtain a cleaned-up manually-segmented slice through the medical images; and
the plurality of transducer layouts being generated based on the cleaned-up manually-segmented slice through the medical images.

2. The method of claim 1, wherein the manually-segmented slice is automatically cleaned up by splitting a segmented area of the manually-segmented slice into a first portion and a second portion based on gray scale values, the first portion being identified as normal tissue and the second portion being identified as abnormal tissue.

3. The method of claim 2, wherein the segmented area is split based on a threshold adjusted by a user-selectable slider of gray scale values.

4. The method of claim 1, wherein the manually-segmented slice is automatically cleaned up by one or more of smoothing one or more edges of a segmented area of the manually-segmented slice, removing one or more discontinuous segmented areas outside a larger segmented area of the manually-segmented slice, or removing one or more non-segmented areas inside a segmented area of the manually-segmented slice.

5. The method of claim 1, wherein the manually-segmented slice is automatically cleaned up by removing one or more discontinuous segmented areas outside a larger segmented area of the manually-segmented slice, and removing one or more non-segmented areas inside a segmented area of the manually-segmented slice.

6. The method of claim 5, wherein the manually-segmented slice is automatically cleaned up further by splitting a segmented area of the manually-segmented slice into a first portion and a second portion based on gray scale values, the first portion being identified as normal tissue and the second portion being identified as abnormal tissue.

7. The method of claim 1, wherein the one or more user-selectable icons (802) to manually segment the slice comprise a user-selectable icon (804) to autofill a region, a user-selectable icon (806) to select the region without autofill, a user-selectable icon (808) to erase a segmentation, a user-selectable icon (810) to assign a tissue type, or a user-selectable icon (812) to expand a border of the region.

8. A non-transitory processor-readable medium containing a set of instructions thereon for generating at least one transducer layout for delivering tumor treating fields to a subject, wherein, when executed by a processor, the instructions cause the processor to perform the method of claim 1.

9. An apparatus for generating at least one transducer layout for delivering tumor treating fields to a subject, the apparatus including one or more processors (1224; 1402), a display and memory (1226; 1403) accessible by the one or more processors (1224; 1402), the memory (1226; 1403) storing instructions that, when executed by the one or more processors (1224; 1402), cause the apparatus to perform the method of:
presenting on the display one or more user-selectable icons (420, 422) to display a slice through medical images of the subject;
presenting on the display one or more user-selectable icons (802) to manually segment the slice through the medical images to obtain a manually-segmented slice through the medical images;
presenting on the display a user-selectable icon (416) to generate a plurality of transducer layouts for application of tumor treating fields to the subject;
**characterized by**:
the medical images being magnetic resonance imaging (MRI) medical images and computer tomography (CT) medical images registered together, wherein the slice comprises corresponding slices through the MRI medical images and the CT medical images overlaid on each other, and the medical images comprise voxels;
presenting on the display a user-selectable icon (814) to automatically clean up the manually-segmented slice to obtain a cleaned-up manually-segmented slice through the medical images; and
the plurality of transducer layouts being generated based on the cleaned-up manually-segmented slice through the medical images.

10. The apparatus of claim 9, wherein the manually-segmented slice is automatically cleaned up by splitting a segmented area of the manually-segmented slice into a first portion and a second portion based on gray scale values, the first portion being identified as normal tissue and the second portion being identified as abnormal tissue.

11. The apparatus of claim 10, wherein the segmented area is split based on a threshold adjusted by a user-selectable slider of gray scale values.

12. The apparatus of claim 9, wherein the manually-segmented slice is automatically cleaned up by one or more of smoothing one or more edges of a segmented area of the manually-segmented slice, removing one or more discontinuous segmented areas outside a larger segmented area of the manually-segmented slice, or removing one or more non-segmented areas inside a segmented area of the manually-segmented slice.

13. The apparatus of claim 9, wherein the manually-segmented slice is automatically cleaned up by removing one or more discontinuous segmented areas outside a larger segmented area of the manually-segmented slice, and removing one or more non-segmented areas inside a segmented area of the manually-segmented slice.

14. The apparatus of claim 13, wherein the manually-segmented slice is automatically cleaned up further by splitting a segmented area of the manually-segmented slice into a first portion and a second portion based on gray scale values, the first portion being identified as normal tissue and the second portion being identified as abnormal tissue.

15. The apparatus of claim 9, wherein the one or more user-selectable icons (802) to manually segment the slice comprise a user-selectable icon (804) to autofill a region, a user-selectable icon (806) to select the region without autofill, a user-selectable icon (808) to erase a segmentation, a user-selectable icon (810) to assign a tissue type, or a user-selectable icon (812) to expand a border of the region.

## Patentansprüche

1. Computerimplementiertes Verfahren zum Erzeugen mindestens eines Wandlerlayouts zum Abgeben von Tumorbehandlungsfeldern an einen Patienten, wobei das Verfahren Folgendes umfasst:
Darstellen eines oder mehrerer von einem Benutzer auswählbarer Symbole (420, 422) auf einer Anzeige zum Anzeigen eines Schnitts durch medizinische Bilder des Patienten;
Darstellen eines oder mehrerer von einem Benutzer auswählbarer Symbole (802) auf der Anzeige, um den Schnitt durch die medizinischen Bilder manuell zu segmentieren, um einen manuell segmentierten Schnitt durch die medizinischen Bilder zu erhalten;
Darstellen eines von einem Benutzer auswählbaren Symbols (416) auf der Anzeige, um eine Vielzahl von Wandlerlayouts zur Anwendung von Tumorbehandlungsfeldern auf den Patienten zu erzeugen;
**dadurch gekennzeichnet, dass**:
die medizinischen Bilder medizinische Magnetresonanztomographie-Bilder (MRT-Bilder) und medizinische Computertomographie-Bilder (CT-Bilder) sind, die miteinander registriert sind, wobei der Schnitt entsprechende Schnitte durch die medizinischen MRT-Bilder und die medizinischen CT-Bilder umfasst, die übereinandergelegt sind, und die medizinischen Bilder Voxel umfassen;
ein von einem Benutzer auswählbares Symbol (814) auf der Anzeige zum automatischen Bereinigen des manuell segmentierten Schnitts dargestellt wird, um einen bereinigten manuell segmentierten Schnitt durch die medizinischen Bilder zu erhalten; und
die Vielzahl von Wandlerlayouts basierend auf dem bereinigten, manuell segmentierten Schnitt durch die medizinischen Bilder erzeugt wird.

2. Verfahren nach Anspruch 1, wobei der manuell segmentierte Schnitt durch Unterteilen eines segmentierten Bereichs des manuell segmentierten Schnitts in einen ersten Abschnitt und einen zweiten Abschnitt basierend auf Graustufenwerten automatisch bereinigt wird, wobei der erste Abschnitt als normales Gewebe identifiziert wird und der zweite Abschnitt als abnormales Gewebe identifiziert wird.

3. Verfahren nach Anspruch 2, wobei der segmentierte Bereich basierend auf einem Schwellenwert unterteilt wird, der durch einen von einem Benutzer auswählbaren Schieberegler für Graustufenwerte angepasst wird.

4. Verfahren nach Anspruch 1, wobei der manuell segmentierte Schnitt durch eines oder mehrere von Glätten einer oder mehrerer Kanten eines segmentierten Bereichs des manuell segmentierten Schnitts, Entfernen eines oder mehrerer diskontinuierlicher segmentierter Bereiche außerhalb eines größeren segmentierten Bereichs des manuell segmentierten Schnitts oder Entfernen eines oder mehrerer nicht segmentierter Bereiche innerhalb eines segmentierten Bereichs des manuell segmentierten Schnitts automatisch bereinigt wird.

5. Verfahren nach Anspruch 1, wobei der manuell segmentierte Schnitt durch Entfernen eines oder mehrerer diskontinuierlicher segmentierter Bereiche außerhalb eines größeren segmentierten Bereichs des manuell segmentierten Schnitts und Entfernen eines oder mehrerer nicht segmentierter Bereiche innerhalb eines segmentierten Bereichs des manuell segmentierten Schnitts automatisch bereinigt wird.

6. Verfahren nach Anspruch 5, wobei der manuell segmentierte Schnitt durch Unterteilen eines segmentierten Bereichs des manuell segmentierten Schnitts in einen ersten Abschnitt und einen zweiten Abschnitt basierend auf Graustufenwerten automatisch weiter bereinigt wird, wobei der erste Abschnitt als normales Gewebe identifiziert wird und der zweite Abschnitt als abnormales Gewebe identifiziert wird.

7. Verfahren nach Anspruch 1, wobei das eine oder die mehreren von einem Benutzer auswählbaren Symbole (802) zur manuellen Segmentierung des Schnitts ein von einem Benutzer auswählbares Symbol (804) zum automatischen Ausfüllen eines Bereichs, ein von einem Benutzer auswählbares Symbol (806) zum Auswählen des Bereichs ohne automatisches Ausfüllen, ein von einem Benutzer auswählbares Symbol (808) zum Löschen einer Segmentierung, ein von einem Benutzer auswählbares Symbol (810) zum Zuweisen eines Gewebetyps oder ein von einem Benutzer auswählbares Symbol (812) zum Erweitern einer Grenze des Bereichs umfassen.

8. Nichtflüchtiges, prozessorlesbares Medium, das einen Satz von Anweisungen zum Erzeugen mindestens eines Wandlerlayouts zum Abgeben von Tumorbehandlungsfeldern an einen Patienten enthält, wobei die Anweisungen, wenn sie von einem Prozessor ausgeführt werden, den Prozessor veranlassen, das Verfahren nach Anspruch 1 durchzuführen.

9. Einrichtung zum Erzeugen mindestens eines Wandlerlayouts zum Abgeben von Tumorbehandlungsfeldern an einen Patienten, wobei die Einrichtung einen oder mehrere Prozessoren (1224; 1402), eine Anzeige und einen Speicher (1226; 1403) einschließt, auf die von dem einen oder den mehreren Prozessoren (1224; 1402) zugegriffen werden kann, wobei der Speicher (1226; 1403) Anweisungen speichert, die, wenn sie von dem einen oder den mehreren Prozessoren (1224; 1402) ausgeführt werden, die Einrichtung veranlassen, das folgende Verfahren durchzuführen:
Darstellen eines oder mehrerer von einem Benutzer auswählbarer Symbole (420, 422) auf der Anzeige zum Anzeigen eines Schnitts durch medizinische Bilder des Patienten;
Darstellen eines oder mehrerer von einem Benutzer auswählbarer Symbole (802) auf der Anzeige, um den Schnitt durch die medizinischen Bilder manuell zu segmentieren, um einen manuell segmentierten Schnitt durch die medizinischen Bilder zu erhalten;
Darstellen eines von einem Benutzer auswählbaren Symbols (416) auf der Anzeige, um eine Vielzahl von Wandlerlayouts zur Anwendung von Tumorbehandlungsfeldern auf den Patienten zu erzeugen;
**dadurch gekennzeichnet, dass**:
die medizinischen Bilder medizinische Magnetresonanztomographie-Bilder (MRT-Bilder) und medizinische Computertomographie-Bilder (CT-Bilder) sind, die miteinander registriert sind, wobei der Schnitt entsprechende Schnitte durch die medizinischen MRT-Bilder und die medizinischen CT-Bilder umfasst, die übereinandergelegt sind, und die medizinischen Bilder Voxel umfassen;
ein von einem Benutzer auswählbares Symbol (814) auf der Anzeige zum automatischen Bereinigen des manuell segmentierten Schnitts dargestellt wird, um einen bereinigten manuell segmentierten Schnitt durch die medizinischen Bilder zu erhalten; und
die Vielzahl von Wandlerlayouts basierend auf dem bereinigten, manuell segmentierten Schnitt durch die medizinischen Bilder erzeugt wird.

10. Einrichtung nach Anspruch 9, wobei der manuell segmentierte Schnitt durch Unterteilen eines segmentierten Bereichs des manuell segmentierten Schnitts in einen ersten Abschnitt und einen zweiten Abschnitt basierend auf Graustufenwerten automatisch bereinigt wird, wobei der erste Abschnitt als normales Gewebe identifiziert wird und der zweite Abschnitt als abnormales Gewebe identifiziert wird.

11. Einrichtung nach Anspruch 10, wobei der segmentierte Bereich basierend auf einem Schwellenwert unterteilt wird, der durch einen von einem Benutzer auswählbaren Schieberegler für Graustufenwerte angepasst wird.

12. Einrichtung nach Anspruch 9, wobei der manuell segmentierte Schnitt durch eines oder mehrere von Glätten einer oder mehrerer Kanten eines segmentierten Bereichs des manuell segmentierten Schnitts, Entfernen eines oder mehrerer diskontinuierlicher segmentierter Bereiche außerhalb eines größeren segmentierten Bereichs des manuell segmentierten Schnitts oder Entfernen eines oder mehrerer nicht segmentierter Bereiche innerhalb eines segmentierten Bereichs des manuell segmentierten Schnitts automatisch bereinigt wird.

13. Einrichtung nach Anspruch 9, wobei der manuell segmentierte Schnitt durch Entfernen eines oder mehrerer diskontinuierlicher segmentierter Bereiche außerhalb eines größeren segmentierten Bereichs des manuell segmentierten Schnitts und Entfernen eines oder mehrerer nicht segmentierter Bereiche innerhalb eines segmentierten Bereichs des manuell segmentierten Schnitts automatisch bereinigt wird.

14. Einrichtung nach Anspruch 13, wobei der manuell segmentierte Schnitt durch Unterteilen eines segmentierten Bereichs des manuell segmentierten Schnitts in einen ersten Abschnitt und einen zweiten Abschnitt basierend auf Graustufenwerten automatisch weiter bereinigt wird, wobei der erste Abschnitt als normales Gewebe identifiziert wird und der zweite Abschnitt als abnormales Gewebe identifiziert wird.

15. Einrichtung nach Anspruch 9, wobei das eine oder die mehreren von einem Benutzer auswählbaren Symbole (802) zur manuellen Segmentierung des Schnitts ein von einem Benutzer auswählbares Symbol (804) zum automatischen Ausfüllen eines Bereichs, ein von einem Benutzer auswählbares Symbol (806) zum Auswählen des Bereichs ohne automatisches Ausfüllen, ein von einem Benutzer auswählbares Symbol (808) zum Löschen einer Segmentierung, ein von einem Benutzer auswählbares Symbol (810) zum Zuweisen eines Gewebetyps oder ein von einem Benutzer auswählbares Symbol (812) zum Erweitern einer Grenze des Bereichs umfassen.

## Revendications

1. Procédé mis en œuvre par ordinateur permettant de générer au moins une disposition de transducteurs pour la délivrance de champs de traitement de tumeur à un sujet, le procédé comprenant :
affichage, sur un écran, d'une ou plusieurs icônes sélectionnables par l'utilisateur (420, 422) pour afficher une coupe à travers des images médicales du sujet ;
affichage, sur l'écran, d'une ou plusieurs icônes sélectionnables par l'utilisateur (802) pour segmenter manuellement la coupe à travers les images médicales afin d'obtenir une coupe segmentée manuellement à travers les images médicales ;
affichage, sur l'écran, d'une icône sélectionnable par l'utilisateur (416) pour générer une pluralité de dispositions de transducteurs pour l'application de champs de traitement de tumeur au sujet ;
**caractérisé par** :
les images médicales étant des images médicales d'imagerie par résonance magnétique (IRM) et des images médicales de tomodensitométrie (TDM) enregistrées ensemble, dans lequel la coupe comprend des coupes correspondantes à travers les images médicales IRM et les images médicales TDM superposées les unes sur les autres, et les images médicales comprennent des voxels ;
affichage, sur l'écran, d'une icône sélectionnable par l'utilisateur (814) pour nettoyer automatiquement la coupe segmentée manuellement afin d'obtenir une coupe segmentée manuellement nettoyée à travers les images médicales ; et
la pluralité de dispositions de transducteurs étant générée sur la base de la coupe segmentée manuellement nettoyée à travers les images médicales.

2. Procédé selon la revendication 1, dans lequel la coupe segmentée manuellement est automatiquement nettoyée en divisant une zone segmentée de la coupe segmentée manuellement en une première portion et une seconde portion sur la base de valeurs de niveaux de gris, la première portion étant identifiée comme tissu normal et la seconde portion étant identifiée comme tissu anormal.

3. Procédé selon la revendication 2, dans lequel la zone segmentée est divisée sur la base d'un seuil ajusté par un curseur de valeurs de niveaux de gris sélectionnable par l'utilisateur.

4. Procédé selon la revendication 1, dans lequel la coupe segmentée manuellement est automatiquement nettoyée par un ou plusieurs parmi le lissage d'un ou plusieurs bords d'une zone segmentée de la coupe segmentée manuellement, la suppression d'une ou plusieurs zones segmentées discontinues à l'extérieur d'une zone segmentée plus grande de la coupe segmentée manuellement, ou la suppression d'une ou plusieurs zones non segmentées à l'intérieur d'une zone segmentée de la coupe segmentée manuellement.

5. Procédé selon la revendication 1, dans lequel la coupe segmentée manuellement est automatiquement nettoyée en supprimant une ou plusieurs zones segmentées discontinues à l'extérieur d'une zone segmentée plus grande de la coupe segmentée manuellement, et en supprimant une ou plusieurs zones non segmentées à l'intérieur d'une zone segmentée de la coupe segmentée manuellement.

6. Procédé selon la revendication 5, dans lequel la coupe segmentée manuellement est automatiquement nettoyée davantage en divisant une zone segmentée de la coupe segmentée manuellement en une première portion et une seconde portion sur la base de valeurs de niveaux de gris, la première portion étant identifiée comme tissu normal et la seconde portion étant identifiée comme tissu anormal.

7. Procédé selon la revendication 1, dans lequel les une ou plusieurs icônes sélectionnables par l'utilisateur (802) pour segmenter manuellement la coupe comprennent une icône sélectionnable par l'utilisateur (804) pour remplir automatiquement une région, une icône sélectionnable par l'utilisateur (806) pour sélectionner la région sans remplissage automatique, une icône sélectionnable par l'utilisateur (808) pour effacer une segmentation, une icône sélectionnable par l'utilisateur (810) pour attribuer un type de tissu, ou une icône sélectionnable par l'utilisateur (812) pour étendre une bordure de la région.

8. Support non transitoire lisible par processeur contenant un ensemble d'instructions sur celui-ci permettant de générer au moins une disposition de transducteurs pour délivrer des champs de traitement de tumeur à un sujet, dans lequel, lorsqu'elles sont exécutées par un processeur, les instructions amènent le processeur à exécuter le procédé selon la revendication 1.

9. Appareil permettant de générer au moins une disposition de transducteurs pour délivrer des champs de traitement de tumeur à un sujet, l'appareil incluant un ou plusieurs processeurs (1224 ; 1402), un écran et une mémoire (1226 ; 1403) accessibles par les un ou plusieurs processeurs (1224 ; 1402), la mémoire (1226 ; 1403) stockant des instructions qui, lorsqu'elles sont exécutées par les un ou plusieurs processeurs (1224 ; 1402), amènent l'appareil à exécuter le procédé de :
affichage, sur l'écran, d'une ou plusieurs icônes sélectionnables par l'utilisateur (420, 422) pour afficher une coupe à travers des images médicales du sujet ;
affichage, sur l'écran, d'une ou plusieurs icônes sélectionnables par l'utilisateur (802) pour segmenter manuellement la coupe à travers les images médicales afin d'obtenir une coupe segmentée manuellement à travers les images médicales ;
affichage, sur l'écran, d'une icône sélectionnable par l'utilisateur (416) pour générer une pluralité de dispositions de transducteurs pour l'application de champs de traitement de tumeur au sujet ;
**caractérisé par** :
les images médicales étant des images médicales d'imagerie par résonance magnétique (IRM) et des images médicales de tomodensitométrie (TDM) enregistrées ensemble, dans lequel la coupe comprend des coupes correspondantes à travers les images médicales IRM et les images médicales TDM superposées les unes sur les autres, et les images médicales comprennent des voxels ;
affichage, sur l'écran, d'une icône sélectionnable par l'utilisateur (814) pour nettoyer automatiquement la coupe segmentée manuellement afin d'obtenir une coupe segmentée manuellement nettoyée à travers les images médicales ; et
la pluralité de dispositions de transducteurs étant générée sur la base de la coupe segmentée manuellement nettoyée à travers les images médicales.

10. Appareil selon la revendication 9, dans lequel la coupe segmentée manuellement est automatiquement nettoyée en divisant une zone segmentée de la coupe segmentée manuellement en une première portion et une seconde portion sur la base de valeurs de niveaux de gris, la première portion étant identifiée comme tissu normal et la seconde portion étant identifiée comme tissu anormal.

11. Appareil selon la revendication 10, dans lequel la zone segmentée est divisée sur la base d'un seuil ajusté par un curseur de valeurs de niveaux de gris sélectionnable par l'utilisateur.

12. Appareil selon la revendication 9, dans lequel la coupe segmentée manuellement est automatiquement nettoyée par un ou plusieurs parmi le lissage d'un ou plusieurs bords d'une zone segmentée de la coupe segmentée manuellement, la suppression d'une ou plusieurs zones segmentées discontinues à l'extérieur d'une zone segmentée plus grande de la coupe segmentée manuellement, ou la suppression d'une ou plusieurs zones non segmentées à l'intérieur d'une zone segmentée de la coupe segmentée manuellement.

13. Appareil selon la revendication 9, dans lequel la coupe segmentée manuellement est automatiquement nettoyée en supprimant une ou plusieurs zones segmentées discontinues à l'extérieur d'une zone segmentée plus grande de la coupe segmentée manuellement, et en supprimant une ou plusieurs zones non segmentées à l'intérieur d'une zone segmentée de la coupe segmentée manuellement.

14. Appareil selon la revendication 13, dans lequel la coupe segmentée manuellement est automatiquement nettoyée davantage en divisant une zone segmentée de la coupe segmentée manuellement en une première portion et une seconde portion sur la base de valeurs de niveaux de gris, la première portion étant identifiée comme tissu normal et la seconde portion étant identifiée comme tissu anormal.

15. Appareil selon la revendication 9, dans lequel les une ou plusieurs icônes sélectionnables par l'utilisateur (802) pour segmenter manuellement la coupe comprennent une icône sélectionnable par l'utilisateur (804) pour remplir automatiquement une région, une icône sélectionnable par l'utilisateur (806) pour sélectionner la région sans remplissage automatique, une icône sélectionnable par l'utilisateur (808) pour effacer une segmentation, une icône sélectionnable par l'utilisateur (810) pour attribuer un type de tissu, ou une icône sélectionnable par l'utilisateur (812) pour étendre une bordure de la région.
